# EUROPEAN PATENT APPLICATION

(11) **EP 4 400 084 A2**
(43) Date of publication of application: **17.07.2024**
(21) Application number: 24020012.1
(22) Date of filing: 09.01.2024
(51) Int. Cl.: A61F 2/50, A61F 2/68, A61F 5/01, H04R 11/00, A61F 2/58

(54) **BIONIC LIMB SYSTEM**

(30) Priority: 10.01.2023 US 202318095466
(71) Applicant: Podhola, Kamil, 46311 Liberec 30 (CZ)
(72) Inventor: Podhola, Kamil, 46311 Liberec 30 (CZ)

(57) **Abstract**

The invention relates to a prosthesis, orthosis or exoskeleton device (a bionic limb system device) for restoring lost functionality to a patient that suffers from at least partial loss of a limb, the device comprising a sound sensing system that communicates with a controller to generate a signal to control the device which can include a defined sensing element, a defined type of a sensor, of a communication interface and/or which can be insulated and/or wherein a controller can command an actuator and/or set a defined parameter. The device can be controllable by a defined sound; it can be coupled with an electrocomponent and/or mechanocomponent. A prosthesis, orthosis or exoskeleton device control method and a sonometry method are proposed.

## Description

This application claims the benefit and priority of Application No. 18,095,466, filed 10 January 2023 (10-01-2023) with U.S.P.T.O. and is hereby incorporated by reference in its entirety.

### Technical Field

The invention relates to a bionic limb system controlled by a human voice.

### Background Art

Recent advances in prosthetic limbs include the provision of multiple degrees of freedom as well as powered actuators that have the potential to provide substantially greater functionality than passive devices. Today's commercially available, upper-extremity prosthetic controllers make uses of the EMG activity (electro-myographic activity generated by muscle contraction) of functional native muscles that are present in the amputee's residual limb. The neurally controlled EMG commands are intuitive and easy to master. In the lower extremity, powered ankle and knee joints are becoming available to the population. Lower-extremity prostheses do not utilize EMG. There is a need to provide smooth, simultaneous volitional-neural control over several degrees of freedom, such as the knee, ankle and subtalar joints. Multiple sources of independent, reliable, and intuitive control can be best obtained by interfacing with the amputee's extrinsic neural control. A Targeted Muscle Re-innervation (TMR) technique can be used for achieving simultaneous control of mutliple prosthetic joints. E.g. four recorded mucle signals can be assigned to prosthetic elbow, wrist, and hand functions. The operator's brain can perform the coordination of the prosthesis joints when a complex task is performed. Inconsistent signal amplitudes and a channel crostalk can occur using EMG technique. A method is provided of restoring at least partial function of a limb, to reversing motor impairment of the limb, to simulating proprioceptive sensory feedback from a device, and to simulate cutaneous sensory feedback from a device. At least one muscle can be selected and transplanted into a remaining biological body and contacted (or associated nerve) with an electrode linked with a device such as a prosthetic limb, an orthotic limb and an exoskeletal limb. The electrodes can be small button-shaped (or needle like, mico-electrodes, or fine wire or conductive polymer filaments sawn into the nerve, or sieve type desing regeneration electrodes in a form of disks of a defined thickness with an array of fine caliber holes or slots drilled through them, etc.) and mounted within a shell of the prosthesis socket so that they can contact the skin which can be also covered with an array of electrodes (surface recording can have various limitations such as signal contamination from activity in neighboring muscles, signal variability caused by perspiration, thicknes of the subcutaneous fat, movement of the electrodes relative to the recorded muscle due to the rotation of the limb and stretching of the skin). Other electrodes materials can be conductive polymers, metal-impregnated woven fabrics, carbon composites, etc. A patch of sking can be translocated onto a non-anatomical portion and contacted with an external prosthetic socket of a prosthetic limb; the patch of skin can be contacted with a nerve cuff linked with a controller actuating the nerves which can include a sensory nerve (e.g. sural, saphenous, tibial, peroneal, median, ulnar, radial nerve). The electrode can be implanted on the epimysium of the muscle or intramuscualrly. Stimulating electrodes can be electrically connected to a motor controller of a device and sensing electrodes can be electrically connected to a sensor controler linked to a sensor of the biological limb (e.g. detecting position, velocity, acceleration, and force of the biological limb) and the sensory controller can transmit information to the motor controller controlling electrical stimulation via the stimulating electrodes. Electrodes can be implanted into paired antagonist muscles. Multiple independent electrical signals, such as electromyographic singals and neural recorded signals can command powered actuators within an external prosthesis. Further artificial sensory information can be provided from the externally controlled lim prosthesis back to the amputee by mechanically stimulating relocated cutaneous tissues salvaged from the amputated limb, or bioelectrically activating sensory nerve fibers in the residual limb using a neural interface. Prostheses can be naturally neurally controlled. Prostheses can include vascular prostheses [e.g. from expanded polytetrafluoroethylene (ePTFE), implantable fabrics such as Dacron can be used; prostheses can include joining different materials, (wound, wrapped, etc.) support structures /e.g. from a biocompatible polymeric material/; components can be from polypropylene, polyethylene, polyurethane, polycarbonate urethane, silicone, acrylate, polynaphtalene, styrene, etc.; metals and alloys can be used; support structures, core structures and various layers, etc.; various fabrication techniques can be used /e.g. molding, jointing, coextrusion, bonding, heating, melting, casting, spraying, 3D printing, etc./; the structures used can have various shapes /e.g. flat, square, ribbon, spherical, elliptical, 2D shapes and 3D forms, etc.]. A cutaneous sensory feedback and proprioceptive feedback (through a direct activation of muclse and tendon afferent nerve fiebers using a (multiple) microchannel array) can be provided. The regenerated nerves can be stimulated with afferent sensory information using stimulating electrodes within a plurality of afferet microchannels of teh microchannel array. The sensing electrodes can be electrically connected to a motor controller of a device. The stimulationg electordes can be electrically connected to a sensory controller of the device. Sensors are stand-alone devices capable of recording electrical activity by means of electrode contacts. Each sensor can be addressble so that registered data can be telemetred to a central receiver terminal located in a shell of the prosthesis. Power for implanted sensors can be supplied via na RF link from a transmitter coil. Implantable Myoelectric Sensors (IMES) systems can be used to measure and transmit the EMG signals from native and/or relocated muscles.

There is a need to provide a human voice controlled prosthesis, orthosis or exoskeleton device (a bionic limb system device).

### Disclosure of Invention

The object of the present invention is to propose a prosthesis, orthosis or exoskeleton device (a bionic limb system device) for restoring lost functionality to a patient that suffers from at least partial loss of a limb, the device characterised in that it comprises: a sound sensing system that communicates with a controller to generate a signal to control the prosthesis, orthosis or exoskeleton device.

It is another object of the present invention to provide the bionic limb system device including a defined sensing element inclusive of a sound sensing coil with a pole area pad.

It is another object of the present invention to provide the bionic limb system device with components provided with a defined type of an insulation.

It is another object of the present invention to provide the bionic limb system device with the controller commanding an actuator and/or setting a defined parameter.

It is another object of the present invention to provide the bionic limb system device further comprising a defined type of a sensor and/or a communication interface.

It is another object of the present invention to provide the bionic limb system device controlled by a defined sound.

It is another object of the present invention to provide the bionic limb system device coupled with a defined electrocomponent and/or mechanocomponent.

It is still another object of the present invention to provide a prosthesis, orthosis or exoskeleton device control method, the method comprising the steps of: providing a prosthesis, orthosis or exoskeleton device for restoring lost functionality to a patient that suffers from at least partial loss of a limb, the device comprising a sound sensing system that communicates with a controller to generate a signal to control said prosthesis, orthosis or exoskeleton device; giving one or more acoustic commands; executing said one or more voice commands by means of the prosthesis, orthosis or exoskeleton device to perform one or more movements, wherein the steps can be repeated.

It is still another object of the present invention to provide a control method in a prosthesis, orthosis or exoskeleton device for restoring lost functionality to a patient that suffers from at least partial loss of a limb, the device comprising a sound sensing system communicating with a controller generating a signal controlling the prosthesis, orthosis or exoskeleton device control method, the method comprising the steps of: measuring contraction or stretching of at least part of the prosthesis, orthosis or exoskeleton device by means of an expanding sensor configured to include an expanding structure wherein a changement in the expanding structure parameters can change a defined resonance frequency and/or harmonics thereof; processing an information from the expanding sensor in a processor to determine a position of said prosthesis, orthosis or exoskeleton device (sonometry method).

It is another object of the present invention to provide the sonometry method with the expanding sensor coupled with an electrocomponent and/or a mechanocomponent; and/or including a movable electrode; and/or forming a sensitive array.

In a first aspect, the invention discloses a prosthesis, orthosis or exoskeleton device for restoring lost functionality to a patient that suffers from at least partial loss of a limb.

In a second aspect, the invention discloses a prosthesis, orthosis or exoskeleton device control method.

In a third aspect, the invention discloses a sonometry method.

### Brief Description of Drawings

The invention will now be described by way of example. Only essential elements of the invention are schematically shown and not to scale nor in proportions to facilitate immediate understanding, emphasis being placed upon illustrating the principles of the invention.
FIG. 1 is a schematic side view of a bionic limb system device according to the present invention.
FIG. 2 is a schematic side view of a bionic hand comprising a palm acoustic sensor.
FIG. 3 is a schematic perspective view of a bionic limb system with a nerve cuff.
FIG. 4 is a schematic side view of a bionic forearm comprising an acoustic sensor.
FIG. 5 is a schematic side view of an acoustic module for a bionic limb system device with a plurality of sound sensing coils.
FIG. 6 is a basic electric circuit which can be used in a bionic limb system device.
FIG. 7 is a schematic frontal view of a combined acoustic sensor including sound sensing coils and sound sensing capacitors which can be used in a bionic limb system device.
FIG. 8 is a schematic side view of a sound sensing coil which can be composed of two opposite wound coils.
FIG. 9 is a schematic oblique view of a pair of opposite wound sound sensing coils.
FIG. 10 is a schematic perspective view of a sound sensing coil with a pole area pad and thermally insulated.
FIG. 11 is a schematic perspective view of a subdimensioned sound sensing coil.
FIG. 12 is a schematic perspective view of a bionic limb system device with an acoustic sensor and attached to a patient's body.
FIG. 13 is a schematic side view of a bionic forearm comprising a string of acoustic sensors.
FIG. 14 is a schematic side view of a sound sensing capacitor which can be used in a bionic limb system device.
FIG. 15 is a schematic side view of a sound sensing optical system which can be provided in a bionic limb system device.
FIG. 16 is a schematic oblique view of a diaphragm which can be provided in a bionic limb system device.
FIG. 17 is a schematic side sectional view of an optical acoustic sensor which can be provided in a bionic limb system device.
FIG. 18 is a schematic side view of a sound sensor which can be provided in a bionic limb system device.
FIG. 19 is a schematic perspective view of a first step of a prosthesis, orthosis or exoskeleton device control method.
FIG. 20 is a schematic oblique view of a first step of a prosthesis, orthosis or exoskeleton device control method.
FIG. 21 is a perspective view of of a bionic limb system device comprising an insulation against moisture, dirt and aseptic environment.
FIG. 22 is a schematic perspective view of a hydrophone which can be provided in a bionic limb system device.
FIG. 23 is a schematic perspective view of an antenna sound sensor which can be provided in a bionic limb system device.
FIG. 24 is a block diagram of a prosthesis, orthosis or exoskeleton device control method.
FIG. 25 is a schematic perspective view of a capacitive sound sensor which can be provided in a bionic limb system device.
FIG. 26 is a schematic side view of an inductive sound sensor which can be provided in a bionic limb system device.
FIG. 27 is a schematic side sectional view of an optical acoustic sensor can be provided in a bionic limb system device.
FIG. 28 is a schematic plan view of an array of sensors forming a matrix which can be provided in a bionic limb system device.
FIG. 29 is a schematic oblique view of an array of sensors which can form an acoustic string and which can be provided in a bionic limb system device.
FIG. 30 is a schematic oblique view of an expanding acoustic sensor provided in a sonometry method used in a bionic limb system device.
FIG. 31 is a schematic side view of an expanding inductive sound sensor which can be provided in a bionic limb system device.
FIGs. 32a and 32b are schematic frontal and side views of a an expanding sound sensor.
FIG. 33 is a schematic plan view of an array of expanding sound sensors.
FIG. 34 is a schematic oblique view of a sound sensing coil with an U core.

### Best Mode for Carrying Out the Invention

The following detailed description shows the best contemplated modes of exemplary embodiments. The description is made for the purpose of illustrating the general principles of the invention, and in such a detail that a skilled person in the art can recognise the advantages of the invention, and can be able to make and use the invention. The objects and advantages of this invention may be realized and obtained as pointed out in the appended claims. Additional advantages may be learned by practice of the invention. The detailed description is not intended to limit the broad principle of the presented invention, but only to show the possibilities of it and to explain the meaning and the sense of the terms used in the appended claims. The description and the detailed description are exemplary and explanatory only.

The terms used in the claims and the specifications shall refer to their synonyms as well.

As used in the claims and the specification, the singular forms are intended to include the plural forms as well and vice versa.

The term "to couple" and derivatives shall refer to a direct or indirect connection via another device and/or connection, such a connection can be mechanical, hydraulical, electrical, electronical, electromagnetical, pneumatical, communication, functional, etc. The connection can be temporary, permantent, detachably attachable, scallable, slotable. Any additional systems may be coupled to the elements, components, etc. and to the system of the invention.

The terms "to comprise", "to include", "to contain", "to provide" and derivatives specify the presence of an element, but do not preclude the presence or addition of one or more other elements or groups and combinations thereof.

The term "consisting of" characterises a Markush group which is by nature closed. Single members of the group are alternatively useable for the purpose of the invention. Therefore, a singular if used in the Markush group would indicate only one member of the group to be used. For that reason are the countable members listed in the plural. That means together with qualifying language after the group "or combinations thereof" that only one member of the Markush group can be chosen or any combination of the listed members in any numbers. In other words, although elements in the Markush groups may be described in the plural, the singular is contemplated as well. Furthermore, the phrase "at least one" preceding the Markush groups is to be interpreted that the group does not exclude one or more additional elements preceded by the phrase.

The invention will be described in reference to the accompanying drawings.

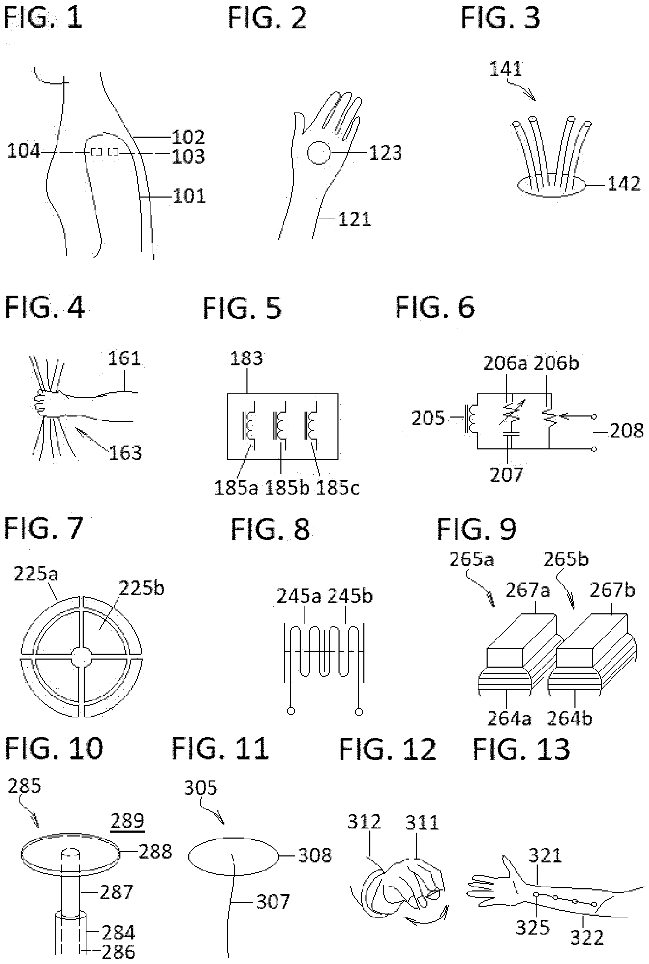

FIG. 1 is a schematic side view of bionic limb system which can be a prosthesis, orthosis or exoskeleton device (101) for restoring lost functionality to a patient (102) that suffers from at least partial loss of a limb, comprising a sound sensing system (103) that communicates with a controller (104) (e.g. having a function of a sensory and/or motor controller) to generate a signal to control said prosthesis, orthosis or exoskeleton device (101). The controller (104) can command an actuator (not shown). The controller (104) can be a centralized processer package having several leads extending from the processor to individual muscles and terminating in electrodes in muscles controlling actuators of a powered prosthesis. The prosthesis, orthosis or exoskeleton device (101) can further comprise a sensor [e.g. a position sensor, a temperature sensor, a biologic functions sensor, an antenna sensor /inclusive of a global positioning antenna sensor/]. A human (or animal) limb can be restored by surgically removing at least a portion of an injured or diseased limb and transplanting a prosthetic limb. At least one pair of agonist and antagonist muscles may be mechanically linked. Electrodes can be inplanted in the muscles and connected to a motor controller. A bionic limb can provide a prosthetic socket including at least one actuator component providing mechanical stimulation of a grafted patch of skin. A bi-directional interface with the peripheral nerves present in the residual limb can be provided (not shown) to control the device (101). Fibers of an amputated nerve may be allowed to grow into a structure of array of micro-channels including motor fibers originally subserving single muscles. Regenerating fibers can grow into either skin tissue or muscle tissue. Signals can be transmitted to and from nerves at the transplanted muscles to control a prosthetic limb (101) linked to the electrodes and extending from a surgical site. Upon regeneration through all the mico-channel arrays, mathematical mapping can be applied, linking recorded sensory afferents from the distal limb and stimulaton efferents to evoke limb muscle activation for stationary and movement patterns, including standing, walking, grasping, etc. The artificial processor (104) can perform computations and can be positioned external to the body and can be worn externally on the patient (102) and implanted neural electronics (not shown). Regenerative Peripheral Nerve Interface (RPNI) can be used providing efferent motor agonist/antagonist signals for the control of the device (101) motors and providing proprioception and cutaneous afferent feedback into peripheral nerves from external prosthetic sensory signals (inclusive of signals from an acoustic sensor). Mechanoreceptors can be used to translate prosthetic sensory information on muscle stretch, tension, and skin pressure and shear into neural signals. Proprioceptive Muscle RPNI (Pro-m-RPNI) method can be used linked in the proposed system adding accoustic signals computing to the known models thereby stimulating proprioceptive sensory feedback from the device (101). The know push-pull system existing on each side of a joint in normal physiology can be mimicked when transferring muscles by placing them in opposition to each other using a mechanical system coupling their movements to each other. I-beam structures can be used in RPNIs. Signals from the acoustic sensors can be used to accomplish the task of mechanically coupled antagonist muscles in Pro-m-RPNI framework allowing feedback from the acoustic sensors which can sense external and/or internal sounds, sounds of the device (101) and/or of the body, sounds of vocal commands (of a therapist, paramedic, of a relative of the patient, etc.). The differential in measured EMG signals can be employed to determine joint state (position/speed) together with acoustic signal patterns which can be compared with recorded acoustic signals (by acoustic sensors). The magnitude in a functional electrical stimulation (FES) can be proportional to the estimated force that would be applied by e.g. wrist flexors prior to limb amputation. Fascicle position and speed sensors can be employed by the Pro-m-RPNI structures together with acoustic sensory information. Sonomicrometry measuring speed of acoustic signals e.g. in muscle tissue and thus the distance between embedded piezoelectric crystals can complete the proposed acoustic system to measure fiber length (contracted or stretched). The external controller can complete information recevied e.g. from a bionic wrist flexed during a handshake by an information recevied from an acoustic sensor (e.g. given by a human voice). In this way an error between the measured bionic wrist position/speed and the measured position/speed from muscle fiber state sensors can be corrected causing the muscle to contract and the antagonist to stretch. The prostetic user can controll himself the position of the prosthetic limb by his voice.

Thus, the known methods for stimulating a proprioceptive sensory organ for a human limb or organ can include acoustic sensors as proposed in the invention. Cutaneous sensory organ for a human limb or organ can be stimulated by means of sensors sensing stress, strain, contact, presure, sheer, etc. on the device. These inputs can be supplemented with an acoustic input from acoustic (mico-) sensors provided at the cutaneous sensory organ and can contribute to simulating a cutaneous sensation at the limb. As known a plurality of Pro-m-RPNI devices can be used to control a single degree of freedom, each device can use its proper acoustic sensing circuit (which can use one or more microchannels). Multiple microprocessors (sensory controllers) can be used to measure signals (inclusive of acoustic) within the bionic limb (101) (e.g signals from a synthetic skin of a prosthetic hand) to convert received singals by the sensory controller (104) into corresponding control command which can be modulated in a closed-loop manner until the measured singal (e.g. muscle strain, one or more measured acoustic singal(s)) is equal to a target tensile skin strain and a target pressure. Skin mechanoreceptors providing natural cutaneous feedback into the nervous sysem and allowing the subject to better manipulate objects can be completed by acoustic sensors providing acoustic feedback for the same reason.

FIG. 2 is a schematic side view of a bionic limb system device (121) comprising an acoustic sensor (123) [which can include a coil and which can be conveniently situated e.g. in a palm and which can be configurated to sense various types of sounds such as a human voice, natural sounds, artificial sounds, etc.].

FIG. 3 is a schematic perspective view of a bionic limb system (141) icluding a nerve cuff (142) which can be linked to a controller and selectively stimulate the native sensory innervation by actuating the nerve cuff (142) with the controller. A closed-loop can be provided between a human and a wearable device (141), sensory information recorded using synthetic sensors on the external device (141) and/or human is mapped to appropriate afferent signals using a micoprocessor located on the device (141) and computing stimulation commands sent to the implanted electronics stimulating nerve cuff (142). Among the other sensors can be one or more sound sensors (acoustic sensors) (not shown) the information of which obtained by the controller (having a microprocessor as a part) can be computed with other sensory information and command the implanted electronics as explained above. Sensory neurons can be used, electrodes implanted on the epimesium of selected muscles or intramuscularly, muscles can be contacted with a mesh array of electrodes, distributed electronics can be used selectively detecting and amplifying signals, innervated skin patches can provide signals, nerve-to-nerve connection can be used. In all these known techniques acoustic sensors can be used to provide information to the system (141). Wired and wireless connections can be used in connection with the controller. The connections can be bidirectional. Microchannel array connections can be used. Neural interfaces can include known immunosuppresants. The controller can modulate afferent signals to transplanted muscles and/or the prosthesis (141). Bi-directional neural interfaces recording efferent motor nerve activity for external control can be used allowing for sensory nerve stimulation from artificial sensors (including acoustic sensors) mounted externally on the device (141) and/or user's body. Stimulating electrodes (not shown) can be connected with a motor controller of the device (141). The sensing electrodes (including acoustic sensors) can be connected to a sensory controller of the device (141). Other sensors can detect position, velocity, acceleration, force, etc. The sensory controller can transmit information to the motor controller stimulating via the stimulating electrodes. Various algorithms generating motor-efferent signals can be used inclusive of algorithms using signals from the acoustic sensors. Various styles of cuff electrodes can be used as developed.

FIG. 4 is a schematic side view of a bionic limb system device (161) comprising an acoustic sensor (163) [which can be configurated to sense various types of sounds such as knocking, tapping, vibrating of a string etc.].

FIG. 5 is a schematic side view of an acoustic module (183) [which can be a transducer] for a bionic limb system device and comprising a plurality of sound sensing coils (185a, 185b, 185c) with cores [which can have symmetrical or unsymmetrical structure]. The acoustic module (183) can convert an acoustic energy [e.g. of a vibrating string, a loudspeakers, a human voice, etc.] into electrical energy by way of electromagnetic induction wherein a changing magnetic field generates a current through wires of the sensing coils (185a, 185b, 185c) which can be wound round pole pieces [or magnetic extensions, fingers, flux conductors, rods, strips, rails, pole pieces with a varying diameter, etc.; and which can extend from the coils (185a, 185b, 185c) symmetrically or unsymmetrically /e.g. which can partially extend from an upper surface with the remainder residing in the interior of coil portion, etc./] and which can be disposed in a magnetic field of permanent magnets [e.g. neodymium iron boron, samarium cobalt, alnico, ceramic or ferrite, etc.]. The output signal from the acoustic module (183) is alternating current (AC). More coils [e.g. two, three or more] with pole pieces and permanent magnets [which can have elongated structure and be transversely polarized; and which can be arranged on opposite sides of a pole piece in various combinations of adjacent south and north poles] can be used having opposite electrical and magnetic polarities and wound in such a way that the current produced by a sound adds up (in-phase), while the current produced by electromagnetic interference in the coils cancels (out-of-phase). A non-magnetic gap [e.g. filled with aluminium, brass, plastic, etc.] or on the contrary an additional ferromagnetic material or spacing may be provided between the permanent magnets and the pole pieces to control the strength of the magnetic field. The coils (185a, 185b, 185c) can be connected in series with the phase relationship as described above. The coils (185a, 185b, 185c) can be insulated and electrostatically shielded (not shown) [e.g. by a copper tape wrapped about the entire assembly of the coils (185a, 185b, 185c), etc.]. A nonmagnetizable housing, a retainer, etc., may be provided.

FIG. 6 is a basic electric circuit which can be used in a bionic limb system device. A transducer (205) provides a voltage and current source, potentiometers (206a, 206b) provide the resistance. A tone potentiometer (206a) can be provided in series with a tone capacitor (207) and a volume potentiometer (206b) [connected as a variable voltage divider] can be coupled with an output connector (208). [Various types of potentiometers can be used such as audio tapers, linear tapers, etc.] Various switches can be provided in the circuit (not shown) to choose a transducer (205) from a plurality of transducers (not shown).

FIG. 7 is a schematic frontal view of a combined acoustic sensor including sound sensing coils (225a) [which can comprise a wire wound round a core] and sound sensing capacitors (225b) which can be used in a bionic limb system device.

FIG. 8 is a schematic side view of a sound sensing coil which can be composed of two opposite wound coils (245a and 245b) with a core [e.g from soft magnetic material and having end and/or intermediate flanges; the cores can be air, soft iron or a permanent magnet, etc.].

FIG. 9 is a schematic oblique view of sound sensing coils (265a, 265b) [which can be substantially of identical construction, e.g. having the same number of effective turns or which can be unbalanced /e.g. having different gauge, diameter, material, impedances, etc./] which can be opposite wound from a common connection [or other cancelling of interfering voltages construction not to be affected by the hum or current induced by adjacent electrical devices such as actuators, electric motors, etc., and at the same time to provide a strong electrical impulse from vibrating pole pieces (267a, 267b] and connected in series or in parallel [which can provide different circuit properties] and include windings (264a, 264b), cores (not shown) [which can be from magnetically permeable materials, magnetizable metals such as soft iron, permanent magnets, alnico 2, 3, 5, ceramic, magnets of different strengths, etc.] and the pole pieces (267a, 267b). [Opposite ends of the cores can be optionally connected with a /relatively small/ permanent magnet preferably transversely magnetized from side to side or electromagnet.]. The windings (264a, 264b) can be connected with electrocomponents (not shown) [e.g. voltage measurements, amplifiers, etc.] to manipulate, amplify, etc. the electrical signals. Variations can be provided such as three and three pole pieces in three separate windings (or different numbers), connected series or parallel and switched in various combinations, slightly offset and overlapped, etc. Modifications can be provided wherein pole pieces can be perpendicular (as shown) or parallel with planes of windings (264a, 264b) or others [e.g. stacked coils]. A continuous bar magnet between the coils (265a, 265b) can be employed instead of pole pieces. More magnets can be employed with various orientations forming a complex magnetic field wherein the coils (265a, 265b) can pickup various components of the field [e.g. in Y and X axes]. Other constructions and components such as (non-magnetic) clamps, screws, bolts, or fasteners, etc., can be further used. The components [i.e. the windings (264a, 264b), the pole pieces (267a, 267b), the cores, etc.] can have various widths, shapes, orientations, etc. Adjustment means can be provided with various degrees of freedom in position. Magnetic flux return path can be provided. Windings (264a, 264b) can be connected with electrocomponents in various ways, can have common terminals, connections between the coils to provide a zero net external voltage due to the field, etc. Physical axes of the coils (265a, 265b) can be generally parallel or may somewhat vary from true parallelism. The coils (265a, 265b) can have common poles, respective pole pieces. The coils (265a, 265b) can be further connected in various signal control circuits [e.g. controlled by a controller with a microprocessor, etc.], amplifiers, noise filters, potentiometers, resistors, (rechargeable) power sources, voltage dividers, compensations, analog-digital converters [which can use various digital communication protocols], etc. At least one coil (265a, 265b) can pickup an ambient noise.

FIG. 10 is a schematic perspective view of a sound sensing coil (285) which can include a winding (284), a core (286) and a pole piece (287) with a pole area pad (288) [preferably from a material suitable for sending and/or receiving flux, sound vibrations, Mylar diaphragm, metal diaphragm attached to a fixed metal plate /e.g. electrostatic condenser technology/ which can be connected to an external power source to charge it; aluminium diaphragm with magnets at either end /e.g. ribbon type technology/, copper foil, brass foil, etc.; the characteristic vibration of the pole area pad (288) can be translated into corresponding variations of electric current flow in the windings (284)] which can be provided on the surface (289) [which can be optionally comprised of a flux repelling material, a non-magnetic material, sound or vibration dampening, inert or amplifying material, etc.] of a bionic limb system device (not shown). The components can be suitably thermally insulated to prevent the device from being scorched. Vibrations can be imparted to the pad (288) directly or via another structure [e.g. an armature].

FIG. 11 is a schematic perspective view of a sound sensing coil (305) which can include a winding, a core, and a pole piece (307) [multiple components can be provided in connective strings with an electronic circuit (not shown)] with a pole area pad (308) [e.g. a magnetic diaphragm]. The embodiment can be provided in a relatively tiny dimensions [e.g. in mili-, micro- or nanometers]. The components can be provided from flexible materials.

FIG. 12 is a schematic perspective view of a bionic limb system device (311) comprising an acoustic sensor (not shown) [which can be positioned at any situation of the device (311)] and attached to a patient's body (312) [e.g. by moving means to a socket, to at elast a portion of selected muscles from a dammaged portion of the human limb, including blood vessels and nerves associated with that portion of the selected muscles, by an attachment to bone in the residual limb, to a multi-chamber silicone structure held in place by sutures to tissuses inclusive of padding the end ot the residual bone and by mechanically shielding related nerve fibers from forces exerted to the exterior surfaces of the residual limb. Other materials and combinations such as laminated structures, can be employed].

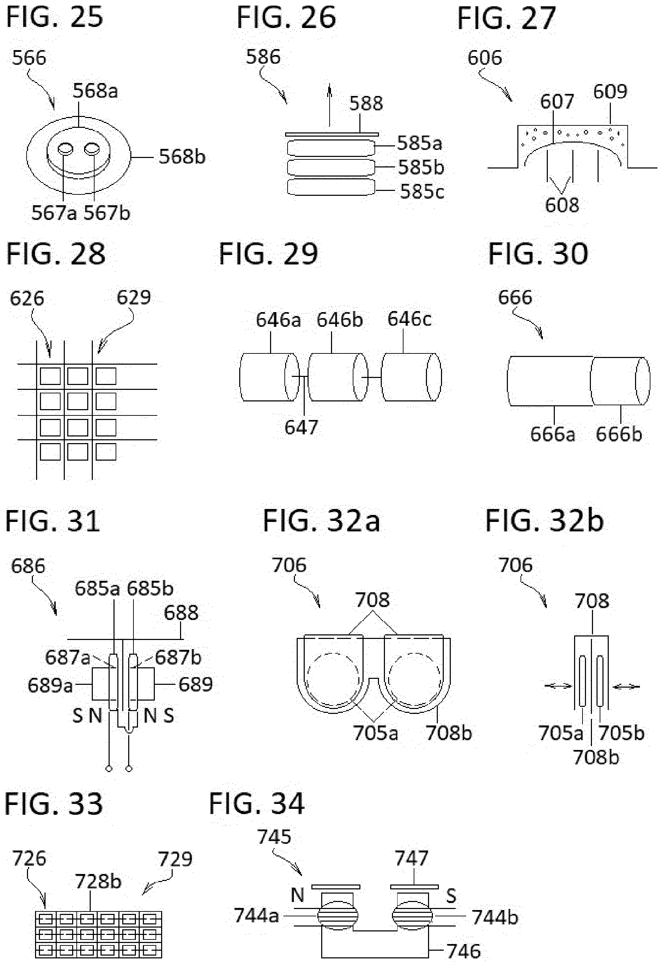

FIG. 13 is a schematic side view of a bionic limb system device (321) comprising an acoustic sensors (325) [which can be positioned in a subcutaneous zone of a patient's forearm (322) and which can be oriented in various ways and which can be expanding acoustic sensors which can be provided in a sonometric system in the proposed bionic limb system /e.g. as shown in FIG. 31/ wherein measurements can be provided in a proximal-distal direction and in other directions /e.g. perpendicular for two or three dimensional measurements].

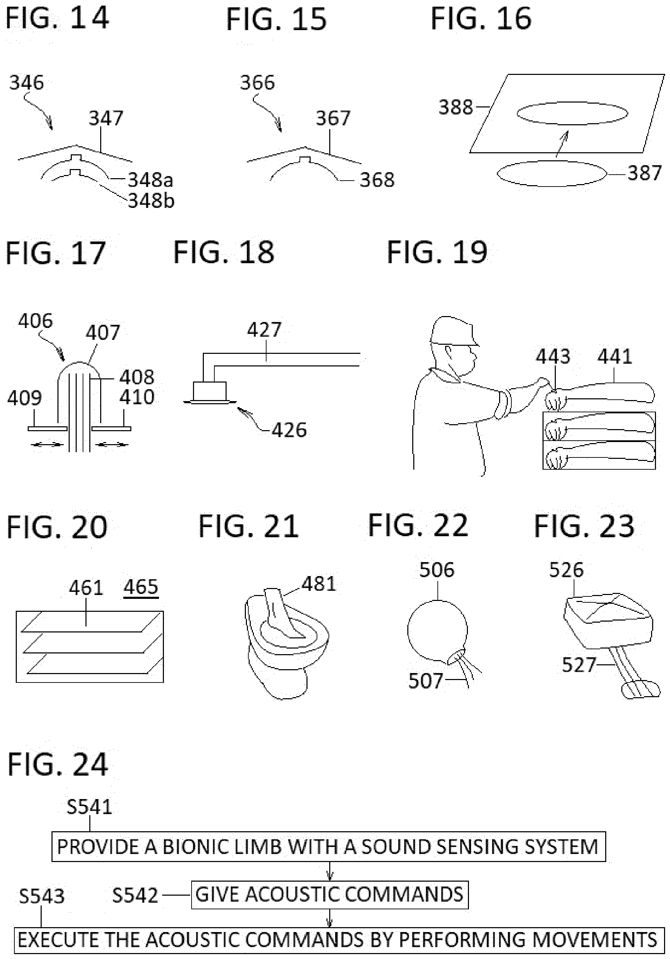

FIG. 14 is a schematic side view of a sound sensing capacitor (346) which can include a diaphragm (347) [which can have at least one preferably inner electrically conductive surface and which can have a various shapes and/or forms], one or more electrically conductive cores (348a, 348b) [which can similarly have various shapes and/or forms and which can be made from aluminium, copper, polysilicon conductive material, etc. and which can be insulated /e.g. by silicon dioxide, etc./]. The space in between can contain an electrically non-conductive layer of air, gas, fluid, foam, etc. The conductive cores (348a, 348b) can be connected with a capacitance sensing circuit and can be charged e.g. via a high resistance from a DC-DC converter forming a charge circuit which can be operated on a pulsed or intermittent basis, or continuously and which can be controlled by a controller. The DC voltage for the converter can be provided from a low-voltage source such as a battery source. The diaphragm (347) can be grounded or charged (polarity is not important but the capacitance of the system in various designs). The signal from either core (348a, 348b) can pass through a filter to a (high impedance) amplifier, an anti-noise signal can be provided to either core (348a, 348b). [Various adaptive or non-adaptive noise cancelling and active noise control methods can be used /e.g. using another microphone, noise sensing transducer, another noise cancelling diaphragm, noise cancelling speakers; fixed, adjustable or adaptive filters/processors; digital and/or analog methods can be used, etc., to detect and/or cancel an ambient noise/.] The core (348b) can be connected with the resistance and the converter, and the core (348a) can be connected with the amplifier. Various multiplate, multicapacitancies configurations can be provided and calculated. The capacitor (346) can be provided in a high-frequency resonant circuit or other capacitance sensing circuits [e.g. it can be connected with an oscillator, to a charging circuit, to a bias voltage, to a digital measurement means, to an analog-digital converter, etc.]. The capacitor (346) can have diphragm (347) and/or either core (248a, 348b) coated with a permanently polarized dielectric material [e.g. electret]. The capacitor (346) can be built on a semiconductor substrate [wherein the cores /electrodes or plates/ comprise aluminium, copper, polysilicon, etc.; wherein an electret material can be deposited as part of a semiconductor fabrication process]. The capacitor (346) can be provided in (shielded) housings including barriers, internal cavities, insulations, acoustical openings [e.g. to eliminate standing waves, resonant effects, aspects of ambient noise, etc.], etc. The barriers can block sound, moisture, humidity, etc. The diaphragm (347) can be placed on a surface of a patient's body and/or a bionic limb system. Components can be provided subcutaneously. The components can be flexible. The diaphragm (347) can be in a direct contact with other components or can be contactless wherein a sensor can detect diaphragm motion in the form of a changing electic, magnetic, electromagnetic fields or an optical signal. The diaphragm (347) can be pressed against a surface and move from an unpressured position to a pressured postion (static displacement). Acoustic waves (20 Hz to 20 kHz) can produce smaller dynamic displacement or vibrations from sub-sonic (5Hz to 20 Hz). Static and dynamic displacements can be used to control sound characteristics of the proposed system. The static displacement can influence a gain or amplitude of the transducer and the time constant and the frequency response. A pressure sensor can be thus constructed using acoustic transducers.

FIG. 15 is a schematic side view of a sound sensing optical system (366) which can include a diaphragm (367) [which can have an inner reflective surface and which can have a various shapes and/or forms], an optical transducer (368).

FIG. 16 is a schematic oblique view of a diaphragm (387) [which can be from a flexible and electrically conductive material, e.g. of a substrate of glass epoxy which can be coated with conductive paint, a vapor aluminium deposition, etc., and coated with a (high dielectric) insulation layer or a dielectric (deformable) substance [e.g. foam, rubber] can be provided in a sandwich type constructions; polycarbonate, Mylar, Ultem, etc., can be used; or a non-conductive or partially conductive diaphragm can be coupled with a conductive diaphragm, etc.] which can be mounted to a mounting (388) [e.g. being made of nylon, Teflon, etc.] to be able to transfer acoustic signals. A sound absorbing material can be used for mounting the diaphragm (387) [e.g. by means of (optionally conductive) rubber] which can be shaped to attenuate acoustic wave from the mounting (388). The diaphragm (387) can be provided with a DC charge with capacitive sound sensing elements [e.g. including a capacitor having one or more capacitive plates or electrodes and providing one or more capacitancies; a space or electrolyte can be provided between the plates, the diaphragm (387), etc.]. The diaphragm (387) [which can be provided with a permanently magnetized material, a ferro-electric layer, adherent or deposited layer which can include a magnetizing coil which can be parallel or normal to the diaphragm (387), a permanent magnet placed in a magnetic interaction, etc.] can be provided with a magnetic charge in inductive sound sensing elements [e.g. including a coil, a printed circuit coil, etc.]. The diaphragm (387) can be provided with a magnetic charge in magnetic sound sensing elements [e.g. including a Hall effect sensor]. The diaphragm (387) can reflect or transfer a light beam [of a laser, visible or infrared light] in optical sound sensing systems [e.g. including an optical-to-electrical transducer, a photodiode, a phototransitor, etc.] or the diaphragm (387) can be coupled with a moving optical element. The diaphragm (387) can by its motion deform a piezoelectric sensor crystal in sound sensing elements with piezo-electric sensors. The diaphragm (387) can transfer [directly or via e.g. a fluid] its movement to sound sensing elements having electro-mechanical sensors. The diaphragm (387) can be electrically insulated from an exterior side and optionally magnetically shielded [e.g. with mu-metals, electrically conductive materials, etc.]. The diaphragm (387) can be coupled in various ways with the mounting (388) [e.g. loosely coupled, adhere to a flexible material, coupled via various types of springs, circumferentially coupled, coupled in coupling points, etc.]. The mounting (388) can be provided of a plurality of elements [e.g. fabricated by bonding together several wafer portions with different patterns and parameters, etching various structures such as channels into the wafers, etc.]. Various types of sound sensing elements can be combined. And various types of sensors can be arranged in opposing relationship (reactive sensors) as far as the generated voltage is concerned to obtain a wider range of sound quality. Variable electrocomponents [e.g. variable capacitors, inductors, rheostats, etc.] can be provided to vary circuits parameters [e.g. a resonant frequency, reactance, impedance, etc.].

FIG. 17 is a schematic side sectional view of an optical acoustic sensor (406) which can comprise a diaphragm (407) [which can comprise a reflective element on either side which can be for example thermally bonded, silicon /which can be fabricated by silicon microfabrication techniques/, silicon nitride, silica, photonic-crystal structure /which can be fabricated by photolitography, silicate bonding, etc./, metallic mirror structure for example with silicon oxide protection and optionally with multiple sublayers from different metals /e.g. aluminium, gold, chromium, silver, etc./, dielectric mirror from silicion dioxide or monoxide, magnesium fluoride, tantalum oxide, etc.; anti-reflection coating on the surface can be provided as well], light reflecting or transmitting structures (408) [e.g. films, fibers such as silica based fibers, cutoff shifted fiber, low-water-peak fiber, dispersion-shifted fiber, non-zero dispersion-shifted fiber, photonic crystal fiber, etc.; or the diaphragm (407) can have a pattern that affects reflectance and which can be printed, etched, etc.; the fibers can be bonded, inserted into a capillary tube, attached by an electric arc, mechanically coupled with the diaphragm (407), tighted with heat-shrink tubing, etc.; the fibers can be considered perfectly smooth, having a scalloping structure, etc.], an emitter (409) and a detector (410). The optical acoustic sensor (406) can convert light signal [visible, infrared, laser light, etc.] to an electric signal [an electronic circuit can provide drive and sensing signals for the emitter (409) and the detector (410) /which can be in various positions/; the change in light signal can be angular or intensity change, linear, pulsating, circular, in various directions, patterns, etc.; optical embodiment can use static and dynamic displacement to provide gain and frequency control]. The acoustic sensor (406) can comprise an optical cavity comprising gas or liquid or a medium with a temperature refractive index change [e.g. a smaller optical cavity can provide more thermal stability]. The sensor (406) can comprise a compressible and elastic element. The transmitting structure (408) can transmit light from a light source such as a monochromatic source [e.g. laser, laser diode], a broadband source [e.g. incadescent lamp, light-emitting diode] and a tunable source [e.g. tunable laser]. A Fabry-Perot sensor with the optical cavity and with optical properties responsive to acoustic waves can be used [e.g. acoustic waves incident on the sensor from the ambient environment or acoustic waves generated within the sensor, etc.] The incident acoustic field can deflect the diafragm and affect the length of the optical cavity and induce a frequency shift in the Fabry-Perot spectrum. A variation in the temperature of the medium in which the sensor (406) is located can also shift the spectrum. Further components can also be provided such as housing and coupling elements having a coefficient of thermal expansion similar to the coefficient of the optical fiber (408) [e.g. comprising silica], fluid conduits, pressure-equalization channels [both in interior and between exterior and interior area], protrusions, holes [wherein a viscous resistance, damping and losses can be taken into account], protective elements [e.g. from polymers], insulation layers, thermally compensating elements provided on the optical fiber (408), on the diaphragm (407), focusing elements such as lens or curved mirrors, sensor fluid reservoirs wherin the Helmhotz resonance can be tailored with the sensor resonance, etc.; gas bubbles can be introduced to the fluid reservoirs to tailor the sensor (406), gas reservoirs can be provided which can be lined by impedance matching layers to suppress standing waves; the normalized (self) noise on the diaphragm (407) can yield a minimum detectable pressure (MDP) which can be tuned to match the minimum ambient noise level, etc. The diaphragm (407) can have various thickness and diameter [a diaphragm fracture strength can limit the maximum pressure on the system; cavitation may occur in liquid filled sensors]; (squeeze-film) damping effects can be reduced by increasing the diameter. The compliance of the diaphragm (407) can determine its displacement as a function of pressure. Thermal expansion of silica (TE), thermally induced variation of the intra-cavity medium refractive index (RIM), and thermally induced variation in the spectral response of the photonic-crystal mirror (PC) can affect the resonance wavelength change with temperature; hydrophones, microphones and various component materials can tailor the system. For hydrophone variants, the ambient fluid can play an important role in the overall mechanics of the acoustic sensor (406) [the fluid can create dissipation, thermal-mechanical noise; to calculate the compliance, the compressibility of the fluid can be taken into account]. The diaphragm (407) of a hydrophone moving in a fluid can radiate part of tis energy into the fluid and create a channel of dissipation. The total harmonic distortion of a sensor (406) in the proposed system can be provided typically as low as possible. An acoustic radiation resistance can be calculated to account for this radiative loss. [The radiation mass and resistance can be calculated by approximating the diaphragm as a rigid piston mounted in an infinite baffle or in certain cases a finite closed baffle, more elaborated baffle models can be used.] The sensors (406) can be placed in arrays, groups, in parallel, etc., wherein the sensors (406) can have various parameters, shared components, etc.

FIG. 18 is a schematic side view of a sound sensor (426) which can be coupled with a circuitry (427) including various electrocomponents (not shown) and provided in the proposed bionic limb system device.

FIG. 19 is a schematic perspective view of a first step of a prosthesis, orthosis or exoskeleton device control method, the step of: providing a prosthesis, orthosis or exoskeleton device (441) for restoring lost functionality to a patient that suffers from at least partial loss of a limb, said device comprising a sound sensing system (443) that communicates with a controller (not shown) [which can be an embodied microchip or an external device communicating wiredly and/or wirelessly] to generate a signal to control said prosthesis, orthosis or exoskeleton device (441).

FIG. 20 is a schematic oblique view of a first step of a prosthesis, orthosis or exoskeleton device control method, the step of: providing a prosthesis, orthosis or exoskeleton device (461) [which can be provided in a freeze box (465)] for restoring lost functionality to a patient that suffers from at least partial loss of a limb, said device comprising a sound sensing system (not shown) that communicates with a controller (not shown).

FIG. 21 is a perspective view of a bionic limb system device (481) comprising an acoustic sensor (not shown) [which can be positioned at any part of the device (481) and which can be watertight and/or provided with an insulation against moisture, dirt, aseptic environment, etc.].

FIG. 22 is a schematic perspective view of a sound sensing element (506) [which can be a hydrophone] which can be couplable via electric leads (507) with various electrocomponents (not shown) of a bionic limb system device (not shown).

FIG. 23 is a schematic perspective view of a sound sensor (526) [which can be an antenna sensor] which can be couplable via electric leads (527) with various electrocomponents (not shown) of a bionic limb system device (not shown).

FIG. 24 is a block diagram of a prosthesis, orthosis or exoskeleton device control method, the method comprising the steps of:
providing a prosthesis, orthosis or exoskeleton device for restoring lost functionality to a patient that suffers from at least partial loss of a limb, said device comprising a sound sensing system that communicates with a controller to generate a signal to control said prosthesis, orthosis or exoskeleton device (S541);
giving one or more acoustic commands (S542);
executing said one or more voice commands by means of said prosthesis, orthosis or exoskeleton device to perform one or more movements (S543),
wherein the steps can be repeated.

FIG. 25 is a schematic perspective view of a sound sensor (566) [which can be a capacitive sensor] which can be couplable via electric leads (567a, 567b) with various electrocomponents (not shown) of a bionic limb system device (not shown). And which can comprise a fixed electrode (568a) and a movable electrode (568b) susceptible to changes in air pressure [and which can be coupled with an (artificial) skin], by which these changes can be converted into voltage changes which can be amplified in a (pre-) amplifier; a drive signal can be frequency or amplitude modulated (FM or AM) [e.g. an alternating current can have a frequency higher than 25 Hz, 50 Hz, 500 Hz or 20 kHz or 44.1 kHz or 10 MHz, etc.]. A DC current can be supplied in another embodiment [e.g. supplied via a coupling capacitor]. The electrodes (568a, 568b) can be connected with signal processing means processing the electrical signal into an analog or digital signal. One electrode can be grounded for electrostatic shielding. A grounded electrostatic shielding of the sensor (566) can be provided (not shown). A second vibration detector can be provided in the proximity (not shown) and connected to the signal processing means [e.g. in opposing phase] and subsequently combined in a differential amplifier [e.g. to reduce the effects of interference and/or stray fields]. A switch can be provided in the circuit. A common housing (not shown) for the components can be provided. The electrical signal can be processed by demodulation, a variable filter, etc. "Hum" transferred to the sensor (566) via a human body can be eliminated by high frequency used in the system. The system can be combined with electromagnetic or other sensing systems [e.g. to avoid crosstalk]. The sensor (566) can be provided by a printed board technique. The electrodes can be made of a conductive material, of a non-conductive material coated with a conductive coating [e.g. paint], a non-conductive electrode can be coupled with a conductive material, etc.

FIG. 26 is a schematic side view of a sound sensor (586) [which can be an inductive sensor] which can be couplable via electric leads (not shown) with various electrocomponents (not shown) of a bionic limb system device (not shown). And which can comprise a movable electrode (588) [which can be a diaphragm from a magnetic material, magnetically permeable material, coupled with a magnetic material, etc.] susceptible to changes in air pressure, by which these changes can be converted into voltage changes which can be amplified in a (pre-) amplifier; a drive signal can be frequency or amplitude modulated (FM or AM) [e.g. an alternating current can have a frequency higher than 25 Hz, 50 Hz, 500 Hz or 20 kHz or 44.1 kHz or 10 MHz, etc.]. A DC current can be supplied in another embodiment [e.g. supplied via a coupling capacitor]. The electrode (588) and one or more (axially spaced) windings (585a, 585b, 585c) [which can be wound in the same or opposite direction but connected in-parallel or in-series in an out-of-phase relationship for cancelling external noises signals, the windings (585a, 585b, 585c) can comprise a bobbin [e.g. of teflon, plastic, fiberglass, paxolin, fiberboard, polycarbonate, etc.; which can have shelves (not shown)]; magnetic polarisations of adjacent magnetic fields /formed by permanent magnets, electromagnets, pole pieces, etc./ can oppose each other; the windings (585a, 585b, 585c) can have a different number of turns, a different gauge, flux conductors of a different permeability to optimize frequency characteristics and EMF noise cancelling; the windings (585a, 585b, 585c) can be tapped or divided; the windings (585a, 585c) can have similar parameters and can be connected in series thus giving a similar output as the winding (585b); these outputs can be connected in-series, in-parallel, in-phase or out-of-phase] can be connected with signal processing means [e.g. a differential amplifier, a converter, etc.] processing the electrical signal into an analog or digital signal. A second vibration detector can be provided in the proximity (not shown) and connected to the signal processing means [e.g. in opposing phase] and subsequently combined in a differential amplifier [e.g. to reduce the effects of interference and/or stray fields]. A switch can be provided in the circuit. A common housing (not shown) for the components can be provided. The electrical signal can be processed by demodulation, a variable filter, etc. "Hum" transferred to the sensor (586) via a human body can be eliminated by high frequency used in the system. The system can be combined with capacitive or other sensing systems [e.g. to avoid crosstalk]. The sensor (586) can be provided by a printed board technique. The electrode (588) can be made of a conductive material, of a non-conductive material coated with a conductive coating [e.g. paint], a non-conductive electrode can be coupled with a conductive material, etc.

FIG. 27 is a schematic side sectional view of an optical acoustic sensor (606) which can comprise a diaphragm (607), light reflecting or transmitting structures (608) [e.g. films, fibers, etc., e.g. three or more than four optical fibers can be used wherein three fibers can lead to a photonic-crystal reflective element of the diaphragm (607), the fourth fiber can be optionally connected to a reference reflective element for calibration purposes; the end of the fibers (608) can be coated with a reflective element; by deforming the diaphragm (607), an incident acoustic signal can modulate the spacing of an optical cavity giving rise to a change in the power of the laser light reflected back into the optical fiber (608)]. The optical acoustic sensor (606) can convert light signal [visible, infrared, laser light, etc.] to an electric signal and can comprise focusing elements such as lens (609) [e.g. plano-concave, bi-concave, minus meniscus; plano-convex, bi-convex, plus meniscus, etc.]. The sensor (606) can be provided on surfaces of a bionic limb device.

FIG. 28 is a schematic plan view of an array of sensors (626) [which can be any type of an acoustic sensor] which can form a matrix (629) characterised by rows and columns [which can be provided in layers insulated by insulating layers and which can comprise a plurality of patterned conductive lines formed by a deposition of a (transparent) material as known in the art; the layers can be disposed between (transparent) substrates (not shown). The matrix (629) can be provided on surfaces of a bionic limb device. The sensors (626) can be localized in a region which can be approximately an order of magnitude smaller than one of the shortest acoustic wavelengths of interest, so that they can be exposed to approximately the same acoustic amplitude or the sensors (626) can be localized in different positions. The sensors (626) can have different parameters [e.g. different diameters, etc.]. Each of the sensors (626) can address a different range of pressures to increase the dynamic range of the array.

FIG. 29 is a schematic oblique view of an array of sensors (646a, 646b, 646c) [which can be any type of an acoustic sensor] which can form a string and which can be connected by acoustic wave leading means (647) [e.g. strings, channels, waveguides etc.]. The array can provide a propagation of the acoustic wave (sound) [e.g. an acoustic communication from a diaphragm (not shown) to a processor, amplifier, etc.].

FIG. 30 is a schematic oblique view of a control method using an expanding acoustic sensor (666) [which can be any type of an acoustic sensor comprising an expanding acoustic structure which can be optionally from more sections (666a, 666b); which can be fabricated from any suitable material such as metals, alloys, polymers, extendable structures, telescopic structures, flexible materials, layered materials, etc., and which can be filled with gas or fluid; which can be provided with sound absorbing and/or sound reflecting layers, etc.] which can have a defined resonance frequency wherein the changement of the expanding structure parameters can change the defined resonance frequency and harmonics thereof. The expanding acoustic sensor (666) can be coupled with a sound capturing diaphragm, with a sound leading system and with a sound processing unit. The sensor (666) can be provided as a sonometric system in the proposed bionic limb system to provide measurements of contraction or stretching the bionic limb structures [such as fibers, muscles, muscular prostheses, etc.], and it can in this way complete the proposed acoustic system. The information from the sensor (666) can be processed in a processor to determine the position of the bionic limb. The expanding sensor (666) can be coupled with various electrocomponents [e.g. electric leads] and mechanocomponents [e.g. acoustic leads, waveguides, etc.].

FIG. 31 is a schematic side view of a an expanding sound sensor (686) [which can be an inductive sensor] which can be couplable via electric leads (not shown) with various electrocomponents (not shown) of a bionic limb system device (not shown). And which can comprise a movable electrode (688) [which can be a diaphragm from a magnetic material, magnetically permeable material, coupled with a magnetic material, an electrically conductive material, etc.] susceptible to changes in air pressure, by which these changes can be converted into voltage changes which can be amplified in a (pre-) amplifier. The movable electrode (688) and one or more (axially spaced) windings (685a, 685b) [which can have similar parameters and which can have opposite windings and be connected in out-of-phase relationship and can have an impedance matching an amplifier, the windings (685a, 685b) can comprise pole pieces (687a, 687b) /e.g. from a ferromagnetic material/; one or more permanent magnets (689a, 689b) can be disposed at one or both ends of the windings (685a, 685b) and direct a magnetic field of a single magnetic polarity into the pole pieces (687a, 687b) from opposite sides thereof; various U, E cores, core plates, slanted structures to concentrate the magnetic field to the pole pieces (687a, 687b), a series of cores, etc., can be provided in place of the permanent magnets (689a, 689b)] can be connected with signal processing means. [The movable electrode (688) can optionally act as an electrostatic shielding between the windings (685a, 685b) to ensure all magnetic coupling.] The shown components can be adjustably mounted to vary circuit parameters. The frequency of the system should be optionally above the range of hearing.

FIGs. 32a and 32b are schematic frontal and side views of a an expanding sound sensor (706) [which can be an inductive sensor] which can be couplable via electric leads (not shown) with various electrocomponents (not shown) of a bionic limb system device (not shown). And which can comprise movable electrodes (708) [which can be for example a metallic diaphragm] susceptible to changes in air pressure, by which these changes can be converted into voltage changes. The movable electrode (708) and one or more (axially spaced) windings (705a, 705b) [which can have a smaller number of turns /inclusive of one turn/ and lesser resistance and inductance or a large number of turns for different applications] can be connected with signal processing means. A fixed electrode (708b) between the windings (705a, 705b) can be grounded to ensure all magnetic coupling. [The expanding sensor (706) can be analogically provided as a capacitive sensor with fixed and movable electrodes.]

FIG. 33 is a schematic plan view of expanding sound sensors (726) which can form an array (729) [e.g. a matrix characterised by rows and columns], which can include a fixed electrode (728b) [which can be formed by a printed board technique and which can be insulated by insulating layers]. The matrix (729) can be provided on surfaces of a bionic limb device and form a sensitive artificial skin which can be sensitive to any mechanical movement [e.g. mechanical waves i.e. caused by a disturbance or vibration in matter, whether solid, gas, liquid /or plasma/ inclusive of sound waves, touch on the skin stimulations, etc.]. The sensors (726) can be localized in any region of a bionic limb (not shown).

FIG. 34 is a schematic oblique view of a sound sensing coil (745) including coil windings (744a, 744b) [which can have for example at least 5,000 turns each and which can be wound in the same direction wherein start ends can be grounded and finish ends can be connected to operational amplifiers /or which can be connected out-of-phase to each other (not shown)/; due to opposite magnetic poles of the different coil windings (744a, 744b), two signals are electrically of opposite polarities; these signals can be processed, wherein an amplifier (including the operational amplifiers) can ad up both signals, and electrically induced noises can be mutually cancelled; other circuit components such as capacitors, resistors, converters, power supplies, etc., can be connected], a core (746) [a shielding can be optionally placed between the poles (not shown) or the gap may be filled with plastic or other dielectric material], and a diaphragm (747) sensitive to changes in air pressure.

No limitations are intended others than as described in the claims. The present invention is not limited to the described exemplary embodiments. It should be noted that various modifications of the bionic limb system can be made without departing from the scope of the invention as defined by the claims.

Elements, integers or components having known equivalents thereof are herein incorporated as if individually set forth.

The material components, materials, chemical substances and compounds, etc., desribed in this specification reflect the state of knowledge at the time of the filling of this application and may be developed in the future.

### Industrial Applicability

The present invention can be used for a large number of applications such as prosthesis, orthosis or exoskeleton devices for restoring lost functionality to a patient which can be a human or an animal. The invention can have application in neural interface technology (e.g. spinal cord lesion patients, stroke patients, motor impairment disabilities). The system can provide an improved functionality of known neuroprostheses, rehabilitation system. The invention can be used in cooperation as a trauma treatment device. Acoustic commands can be given during medical therapy and rehabilitation phase which can make it easier for a patient to regain control of the limb and accelerate the therapy. Acoustic sensors can improve feedback information of the system. Closed-loop control algorithms for restoring biological functions in disability using information from acoustic sensors can be used. The invention can improve motor and sensing applications. The proposed sonometric method can provide a control method for the proposed bionic limb system. The system can be provided in a modular system wherein modules can be modularly scalable and/or exchangeable and can be catalogued.

## Claims

1. A prosthesis, orthosis or exoskeleton device for restoring lost functionality to a patient that suffers from at least partial loss of a limb, said device **characterised in that** it comprises: a sound sensing system that communicates with a controller to generate a signal to control said prosthesis, orthosis or exoskeleton device.

2. The prosthesis, orthosis or exoskeleton device according to claim 1, including a sound sensing element, wherein at least one said sound sensing element is selected from the group consisting of microphones, hydrophones, coils, capacitors, optical systems, Hall effect sensors, piezo-electric sensors, electro-mechanical sensors, antenna sensors, analog sensors, digital sensors, or combinations thereof.

3. The prosthesis, orthosis or exoskeleton device according to claim 2, wherein said sound sensing coil has a pole area pad.

4. The prosthesis, orthosis or exoskeleton device according to claim 1, wherein at least one component is provided with an insulation, wherein at least one said insulation is selected from the group consisting of thermal insulations, electrical insulations, dielectric insulations, magnetic insulations, vibrations insulations, acoustic insulations, insulations against gases, insulations agains liquids, insulations against moisture, insulations against biological influences, insulations against chemicals, insulations against dirt, insulations against aseptic environments, or combinations thereof.

5. The prosthesis, orthosis or exoskeleton device according to claim 1, wherein said controller commands an actuator.

6. The prosthesis, orthosis or exoskeleton device according to claim 1, wherein said controller sets a parameter, wherein at least one said paramether is selected from the group consisting of velocity, force, acceleration, deceleration, action time, pause length, angular velocity, two dimensional motion, three dimensional motion, or combination thereof.

7. The prosthesis, orthosis or exoskeleton device according to claim 1, further comprising a sensor, wherein at least one said sensor is selected from the group consisting of acoustic sensors, electric field potential sensors, position sensors, light sensors, electromagnetic sensors, magnetic sensors, temperature sensors, biologic functions sensors, antenna sensors, or combinations thereof.

8. The prosthesis, orthosis or exoskeleton device according to claim 1, further comprising a communication interface to communicate with an exterior computing system.

9. The prosthesis, orthosis or exoskeleton device according to claim 1, wherein at least one said sound is selected from the group consisting of human voices, sounds emitted by an electronic device, sounds emitted by loud speakers, musical sounds, natural sounds, artificial sounds, or combinations thereof.

10. The prosthesis, orthosis or exoskeleton device according to claim 1, coupled with an electrocomponent, wherein at least one said electrocomponent is selected from the group consisting of signal generators, oscillators, capacitance sensing circuits, inductance sensing circuits, charging circuits, electronic leads, electric leads, receptacles, plugs, interfaces, terminal blocks, pickups, transducers, potentiometers, coils, cores, solenoids, permanent magnets, pole pieces, sensors, targets, actuators, microphones, dynamic microphones, condenser microphones, ribbon microphones, cardioid microphones, bi-directional microphones, optical microphones, emitters, electret microphones, drivers, dynamic drivers, magnetic drivers, electrostatic drivers, headphones, speakers, loud speakers, amplifiers, preamplifiers, transistors, field effect transistors, resonators, rectifiers, filters, inverters, converters, analog-digital converters, transformers, voltage measurements, digital measurement circuits, voltage regulators, compensations, power electronics, controllers, integrated circuits, control circuits, processors, inductors, capacitors, resistors, diodes, varactors, variable electrocomponents, switches, conductors, semiconductors, rechargeable power sources, source management systems, power supplies, electric motors, or combinations thereof.

11. The prosthesis, orthosis or exoskeleton device according to claim 1, coupled with a mechanocomponent, wherein at least one said mechanocomponent is selected from the group consisting of mechanical joints, receptacles, plugs, diaphragms, acoustic leads, waveguides, or combinations thereof.

12. A prosthesis, orthosis or exoskeleton device control method, the method comprising the steps of:
providing a prosthesis, orthosis or exoskeleton device for restoring lost functionality to a patient that suffers from at least partial loss of a limb, said device comprising a sound sensing system that communicates with a controller to generate a signal to control said prosthesis, orthosis or exoskeleton device;
giving one or more acoustic commands;
executing said one or more voice commands by means of said prosthesis, orthosis or exoskeleton device to perform one or more movements,
wherein the steps can be repeated.

13. In a prosthesis, orthosis or exoskeleton device for restoring lost functionality to a patient that suffers from at least partial loss of a limb, said device comprising a sound sensing system communicating with a controller generating a signal controlling said prosthesis, orthosis or exoskeleton device control method, the method comprising the steps of:
measuring contraction or stretching of at least part of said prosthesis, orthosis or exoskeleton device by means of an expanding sensor, said expanding sensor configured to include an expanding structure, wherein a changement in said expanding structure parameters can change a defined resonance frequency and/or harmonics thereof;
processing an information from said expanding sensor in a processor to determine a position of said prosthesis, orthosis or exoskeleton device.

14. The prosthesis, orthosis or exoskeleton device control method according to claim 13, wherein said expanding sensor is coupled with an electrocomponent and/or a mechanocomponent, to enhance a functionality of said prosthesis, orthosis or exoskeleton device.

15. The prosthesis, orthosis or exoskeleton device control method according to claim 13, wherein said expanding sensor includes a movable electrode.
